(19) Europäisches Patentamt
European Patent Office
Office européen des brevets

(11) Veröffentlichungsnummer: **0 176 998**
**B1**

(12) # EUROPÄISCHE PATENTSCHRIFT

(45) Veröffentlichungstag der Patentschrift: **08.11.89**

(21) Anmeldenummer: **85112351.3**

(22) Anmeldetag: **30.09.85**

(51) Int. Cl.[4]: **C 07 D 233/60,** C 07 D 233/61, C 07 D 249/08, A 61 K 31/415, A 61 K 31/41 // C07D405/06, C07D407/06, C07D317/26, C07C49/185

(54) **Antimykotisches Mittel.**

(30) Priorität: **05.10.84 DE 3436452**

(43) Veröffentlichungstag der Anmeldung: **09.04.86 Patentblatt 86/15**

(45) Bekanntmachung des Hinweises auf die Patenterteilung: **08.11.89 Patentblatt 89/45**

(84) Benannte Vertragsstaaten: **AT BE CH DE FR GB IT LI NL SE**

(56) Entgegenhaltungen:
**EP-A- 0 086 917**
**EP-A- 0 097 881**
**EP-A- 0 102 591**
**EP-A- 0 111 146**
**EP-A- 0 111 711**
**EP-A- 0 114 487**
**EP-A- 0 141 204**

(73) Patentinhaber: **BAYER AG, D-5090 Leverkusen 1 Bayerwerk (DE)**

(72) Erfinder: **Holmwood, Graham, Dr., Krutscheider Weg 105, D-5600 Wuppertal 11 (DE)**
Erfinder: **Krämer, Wolfgang, Dr., Am Eckbusch 39/45, D-5600 Wuppertal 1 (DE)**
Erfinder: **Büchel, Karl Heinz, Prof. Dr., Dabringhausener Strasse 42, D-5093 Burscheid (DE)**
Erfinder: **Plempel, Manfred, Dr., Zwengenberger Strasse 3c, D-5657 Haan (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft die Verwendung von Hydroxyethyl-azol-Derivaten als antimikrobielle Mittel, insbesondere als Antimykotika.

Es ist bereits bekannt geworden, dass bestimmte 1-Hydroxyethyl-azolyl-Derivate allgemein gute antimykotische Eigenschaften aufweisen.

So sind aus der EP-A-0 111 146 antimykotisch wirksame 1-Hydroxyethylazole der allgemeinen Formel

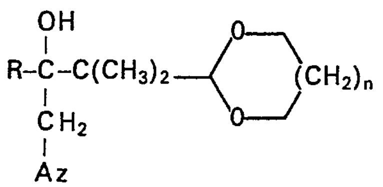

bekannt geworden, in denen R beispielsweise für gegebenenfalls substituiertes Phenyl, Az für 1,2,4-Triazol-1-yl, 1,2,4-Triazol-4-yl oder Imidazol-1-yl und n für 0 oder 1 steht. Wirkspektrum und Wirkstärke dieser Substanzen sind jedoch nicht in allen Indikationen zufriedenstellend.

Es wurde gefunden, dass die Hydroxylethylazol-Derivate der allgemeinen Formel

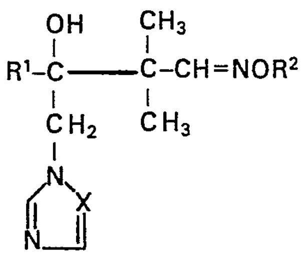

(I)

in welcher

$R^1$ für geradkettiges oder verzweigtes Alkyl mit 1 bis 6 Kohlenstoffatomen oder für die Gruppierung Ar–Y;

Ar für Naphthyl oder für gegebenenfalls einfach oder mehrfach, gleich oder verschieden substituiertes Phenyl, wobei als Substituenten vorzugsweise genannt seien:

Halogen, Alkyl mit 1 bis 4 Kohlenstoffatomen, Alkoxy und Alkylthio mit jeweils 1 bis 2 Kohlenstoffatomen, Nitro, Halogenalkyl und Halogenalkoxy sowie Halogenalkylthio mit jeweils 1 bis 2 Kohlenstoffatomen und 1 bis 5 gleichen oder verschiedenen Halogenatomen, wie vorzugsweise Fluor und Chloratomen, der $-CH=NOR^2$-Rest, sowie jeweils gegebenenfalls durch Halogen und/oder Alkyl mit 1 bis 2 Kohlenstoffatomen substituiertes Phenyl, Phenoxy, Benzyl und Benzyloxy;

X für ein Stickstoffatom oder die CH-Gruppe;

Y für eine direkte Bindung oder für die Gruppierungen $-CH_2-$, $-CH_2CH_2-$, $-OCH_2-$, $-SCH_2-$, $-CH=CH-$ oder $-C\equiv C-$;

und

$R^2$ für Wasserstoff, geradkettiges oder verzweigtes Alkyl mit 1 bis 6 Kohlenstoffatomen, Alkenyl und Alkinyl mit jeweils 2 bis 6 Kohlenstoffatomen, für gegebenenfalls einfach oder mehrfach, gleich oder verschieden substituiertes Phenylalkyl mit 1 bis 2 Kohlenstoffatomen im Alkylteil, wobei als Phenylsubstituenten die bei Ar bereits genannten Phenylsubstituenten infrage kommen; sowie für gegebenenfalls einfach bis dreifach, gleich oder verschieden durch Alkyl mit 1 bis 3 Kohlenstoffatomen substituiertes Cycloalkylmethyl mit 5 oder 6 Kohlenstoffatomen im Cycloalkylteil steht und deren Säureadditions-Salze gute mikrobielle, insbesondere antimykotische Eigenschaften, aufweisen.

Die Verbindungen der Formel (I) besitzen ein asymmetrisches Kohlenstoffatom und können deshalb in den beiden optischen Isomerenformen anfallen.

Überraschenderweise zeigen die erfindungsgemäss zu verwendenden Hydroxyethyl-azol-Derivate der Formel (I) in bestimmten Indikationsbereichen ein besseres Wirkungsspektrum, als die aus dem Stand der Technik bekannten 1-Hydroxyethyl-azolyl-Derivate, wie 1-(4-Chlor-phenoxy)-3,3-dimethyl-2-(imidazol-1-yl-methyl) -2-butanol und 1-(2-Methylphenoxy) -3,3-dimethyl -2-(imidazol -1-yl-methyl) -2-butanol, welches konstitutionell und wirkungsmässig naheliegende Verbindungen sind.

Bevorzugt verwendet werden diejenigen Verbindungen der Formel (I), in denen

$R^1$ für geradkettiges Alkyl mit 1 bis 6 Kohlenstoffatomen oder für die Gruppierung Ar–Y– steht;

Ar für Naphthyl oder für gegebenenfalls einfach bis dreifach, gleich oder verschieden substituiertes Phenyl steht, wobei als Substituenten genannt seien:

Fluor, Chlor, Methyl, Trifluormethyl, Trifluormethoxy, Trifluormethylthio, Methoximinomethyl, Ethoximinomethyl, Allyloximinomethyl sowie jeweils gegebenenfalls durch Chlor und/oder Methyl substituiertes Phenyl, Phenoxy, Benzyl und Benzyloxy;

X für ein Stickstoffatom oder die CH-Gruppe steht;

Y für eine direkte Bindung oder für die Gruppierungen $-CH_2-$, $-CH_2CH_2-$, $-OCH_2-$, $-SCH_2-$, $-CH=CH-$ oder $-C\equiv C-$ steht;

und

$R^2$ für Wasserstoff, Methyl, Ethyl, n-Propyl, n-Butyl, Allyl, Propargyl, für gegebenenfalls einfach bis dreifach, gleich oder verschieden durch Fluor, Chlor, Methyl, Trifluormethyl oder Trifluormethoxy substituiertes Benzyl sowie für gegebenenfalls durch Methyl oder Ethyl substituiertes Cyclohexylmethyl steht.

Bevorzugte erfindungsgemässe Verbindungen sind auch Additionsprodukte aus Säuren und denjenigen Hydroxyethyl-azol-Derivaten der Formel (I), in denen die Substituenten $R^1$, X und Z die Bedeutungen haben, die bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Die erfindungsgemäss zu verwendenden Hydroxyethyl-azol-Derivate sowie ihre Säureadditions-Salze sind noch nicht beschrieben. Sie sind jedoch Gegenstand einer eigenen älteren Patentanmeldung, EP-A-141 204 und können erhalten werden, indem man

a) Hydroxyethylazolyl-acetal-Derivate der Formel (II)

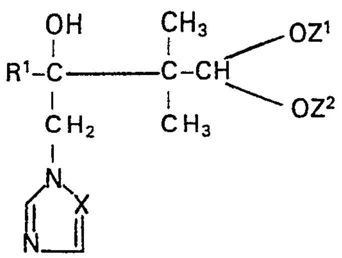

(II)

in welcher

$Z^1$ und $Z^2$ für Alkyl stehen, oder gemeinsam mit den Sauerstoffatomen, an die sie gebunden sind, für einen Dioxolan-Ring stehen; und

$R^1$ und X die oben angegebenen Bedeutungen haben, in Gegenwart eines Gemisches aus Wasser und einem organischen Lösungsmittel, wie beispielsweise Alkohole und in Gegenwart einer Säure, wie beispielsweise Salzsäure, bei Temperaturen zwischen 30 °C und 120 °C umsetzt; oder

b) die nach dem Verfahren (a) erhaltenen Hydroxylethyl-azol-Derivate der Formel (Ia),

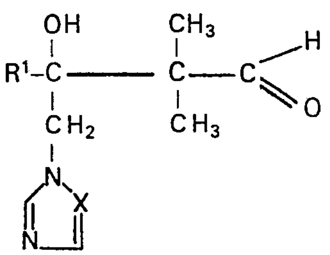

(Ia)

in welcher

$R^1$ und X die oben angegebenen Bedeutungen haben, oder die Hydroxyethylazolyl-acetal-Derivate der Formel (II) mit Hydroxylamin-Derivaten der Formel (III),

$H_2N-O-R^2$ (III)

in welcher

$R^2$ die oben angegebenen Bedeutungen hat, in Gegenwart eines Verdünnungsmittels, wie beispielsweise Alkohole und Wasser, bzw. Gemische beider, bei Temperaturen zwischen 50 °C und 100 °C umsetzt; oder

c) die nach dem Verfahren (b) erhaltenen Hydroxyethyl-azol-Derivate der Formel (Ib) (das sind solche Verbindungen der Formel (I), bei denen $R^2$ für die Gruppe OH steht),

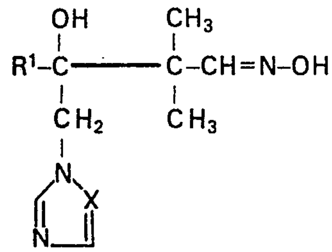

(Ib)

in welcher

$R^1$ und X die oben angegebenen Bedeutungen haben, mit Halogeniden der Formel (IV),

$Hal-R^3$ (IV)

in welcher

Hal für Chlor, Brom oder Jod steht und

$R^3$ für die Bedeutungen von $R^2$, ausser für Wasserstoff, steht, in Gegenwart eines inerten organischen Lösungsmittels, wie beispielsweise Dimethylsulfoxid, und gegebenenfalls in Gegenwart einer starken Base, wie beispielsweise Alkalimetallhydride oder -amide, bei Temperaturen zwischen 60 °C und 100 °C umsetzt; oder

d) Oxirane der Formel (V),

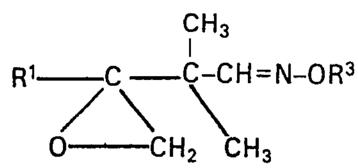

(V)

in welcher

$R^1$ und $R^3$ die oben angegebenen Bedeutungen haben, mit Azolen der Formel (VI),

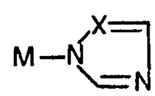

(VI)

in welcher

M für Wasserstoff oder ein Alkalimetall, vorzugsweise Natrium und Kalium steht, in Gegenwart eines inerten organischen Lösungsmittels, wie beispielsweise Alkohole oder Dimethylformamid, und gegebenenfalls in Gegenwart einer Base, wie beispielsweise Natriumalkoholat oder Kaliumhydroxid, bei Temperaturen zwischen 60 °C und 150 °C umsetzt.

An die so erhaltenen Verbindungen der Formel (I) kann gegebenenfalls eine Säure addiert werden.

Die Hydroxyethylazolyl-acetal-Derivate der Formel (II) sind bekannt (vergl. DE-OS 3 242 222 und DE-OS 3 242 252); bzw. lassen sie sich nach dem dort angegebenen Verfahren erhalten, indem man Oxirane der Formel (VII)

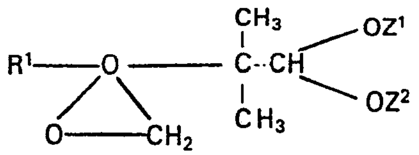

(VII)

in welcher

$R^1$, $Z^1$ und $Z^2$ die oben angegebenen Bedeutungen haben, mit Azolen der Formel (VI) in Gegenwart eines inerten organischen Lösungsmittels, wie beispielsweise Alkohole, und gegebenenfalls in Gegenwart einer Base, wie beispielsweise Natriumalkoholat oder Kaliumhydroxid, bei Temperaturen zwischen 60 °C und 150 °C umsetzt.

Die Oxirane der Formel (VII) sind bekannt (vergl. DE-OS 3 242 252); bzw. können sie in allgemein bekannter Art und Weise erhalten werden, indem man Ketone der Formel (VIII),

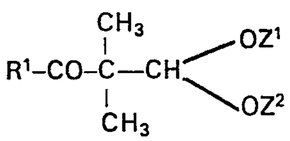

(VIII)

in welcher

$R^1$, $Z^1$ und $Z^2$ die oben angegebene Bedeutung haben, enweder

α) mit Dimethyloxosulfonium-methylid der Formel (IX)

$$\overset{\delta+}{(CH_3)_2SO}\overset{\delta-}{CH_2} \qquad (IX)$$

in an sich bekannter Weise in Gegenwart eines Verdünnungsmittels, wie beispielsweise Dimethylsulfoxid, bei Temperaturen zwischen 20 °C und 80 °C umsetzt (vgl. hierzu die Angaben in J. Am. Chem. Soc. 87, 1363–1364 (1965) ),

oder

β) mit Trimethylsulfonium-methylsulfat der Formel (X)

$$\left[ (CH_3)_3S^{(+)} \right] CH_3SO_4^{(-)} \qquad (X)$$

in an sich bekannter Weise in Gegenwart eines inerten organischen Lösungsmittels, wie z.B. Acetonitril, und in Gegenwart einer Base, wie z.B. Natriummethylat, bei Temperaturen zwischen 0 °C bis 60 °C, vorzugsweise bei Raumtemperatur, umsetzt (vergl. auch die Angaben in Heterocycles 8, 397 (1977) ).

Die erhaltenen Oxirane der Formel (VII) können gegebenenfalls ohne Isolierung direkt weiter umgesetzt werden.

Die Ketone der Formel (VIII) sind teilweise bekannt (vergl. z.B. J. Org. Chem. 32, 404 (1967); DE-OS 3 224 130, DE-OS 3 224 129 und DE-OS 3 242 252); bzw. können sie in bekannter Art und Weise erhalten werden, indem man z.B. 1-(N-Morpholino)-isobuten der Formel (XI)

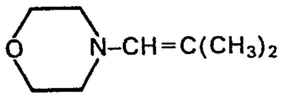

(XI)

mit Chloriden der Formel (XII)

in welcher

$R^1$ die oben angegebene Bedeutung hat, in Gegenwart eines Lösungsmittels, wie beispielsweise Diethylether, bei Temperaturen zwischen 20 °C und 120 °C umsetzt und die so erhaltenen Keto-Derivate der Formel (XIII),

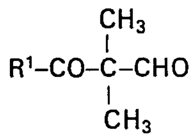

(XIII)

in welcher

$R^1$ die oben angegebene Bedeutung hat, in üblicher Weise an der Aldehydgruppe derivatisiert, wie z.B. mittels Ethylenglykol in Gegenwart eines inerten organischen Lösungsmittels, wie beispielsweise Toluol, und in Gegenwart einer starken Säure als Katalysator, wie beispielsweise p-Toluolsulfonsäure, bei Temperaturen zwischen 80 °C und 110 °C.

In manchen Fällen erweist es sich als vorteilhaft, den Rest $R^1$ bzw. Teile davon erst nach der Derivatisierung an der Aldehydgruppe einzuführen (vergl. auch die Herstellungsbeispiele).

Die Hydroxylamin-Derivate der Formel (III) sind allgemein bekannte Verbindungen der organischen Chemie.

Die Halogenide der Formel (IV) sind allgemein bekannte Verbindungen der organischen Chemie.

Die Oxirane der Formel (V) sind noch nicht bekannt; sie können jedoch in allgemein bekannter Art und Weise erhalten werden, indem man Keto-oxim-Derivate der Formel (XIV),

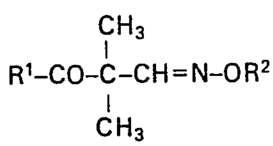

(XIV)

in welcher

$R^1$ und $R^2$ die oben angegebenen Bedeutungen haben, nach den oben angegebenen Verfahren (α) bzw. (β) epoxidiert.

Die Keto-oxim-Derivate der Formel (XIV) sind bekannt (vergl. DE-OS 3 224 130; DE-OS 3 224 129 und DE-OS 3 242 252); bzw. können sie in bekannter Art und Weise erhalten werden, indem man z.B. 1-(N-Morpholino)-isobuten der Formel (XI) mit Chloriden der Formel (XII) in Gegenwart eines Lösungsmittels, wie beispielsweise Diethylether, bei Temperaturen zwischen 20 °C und 120 °C umsetzt und die so erhaltenen Keto-Derivate der Formel (XIII) in üblicher Weise an der Aldehydgruppe mittels Hydroxylamin-Derivaten der Formel (III), wie z.B. Methoxyhydroxylamin-hydrochlorid, in Gegenwart eines inerten organischen Lösungsmittels, wie beispielsweise Ethanol und in Gegenwart von Natriumacetat bei Temperaturen zwischen 80 °C und 110 °C derivatisiert. In manchen Fällen erweist es sich als vorteilhaft, den Rest $R^1$ bzw. Teile davon erst nach der Derivatisierung der Aldehydgruppe einzuführen.

Die Azole der Formel (VI) sind allgemein bekannte Verbindungen der organischen Chemie.

Die Verbindungen der Formel (I) können in Säureadditions-Salze überführt werden.

Die Säureadditions-Salze der Verbindungen der Formel (I) können in einfacher Weise nach üblichen Salzbildungsmethoden, z.B. durch Lösung einer Verbindung der Formel (I) in einem geeigneten inerten Lösungsmittel und Hinzufügen der Säure, z.B. Salzsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemässen verwendbaren Verbindungen der Formel (I), deren Ester-Derivate und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sprosspilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:

Dermatomykosen und Systemmykosen durch Trichophyton mentagrophytes und andere Trichophytonarten, Microsporonarten, Epidermophyton floccosum, Sprosspilze und bi-phasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemässe Wirkstoffe enthalten oder die aus einem oder mehreren erfindungsgemässen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, dass die Zubereitungen in Form einzelner Teile, z.B. Tablette, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulaten können mit den üblichen gegebenenfalls Opakisierungsmittel enthaltenen Überzügen und Hüllen versehen sein und so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonit, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süssmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemässen Wirkstoffe, sowie von pharmazeutischen Zubereitungen, die einen oder mehrere erfindungsgemässe Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemässen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oraler Applikation werden die erfindungsgemässen Wirkstoffe in Gesamtmengen von etwa 2,5 bis etwa 200, vorzugsweise 5 bis 150 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben aufgeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

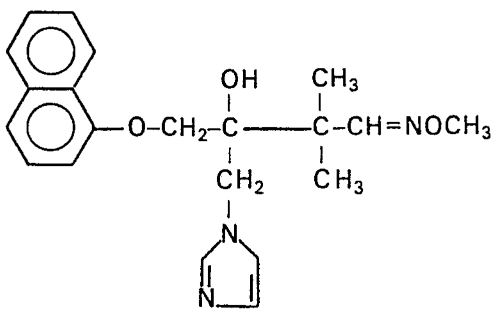

9 g (0,027 Mol) 2,2-Dimethyl -3-hydroxy -3-(imidazol -1-yl-methyl) -4-(naphth -1-oxy)-butanal (Beispiel 2a) und 2,2 g (0,027 Mol) O-Methyl-hydroxylamin-hydrochlorid werden in 60 ml Ethanol 16 Stunden unter Rückfluss erhitzt. Anschliessend wird das Reaktionsgemisch eingeengt, der Rückstand in Petrolether aufgeschlämmt, abgesaugt und getrocknet.

Man erhält 9 g (90,7% der Theorie) 2-(Imidazol-1-yl-methyl) -3-methoximinomethyl -3-methyl -1-(naphth -1-oxy) -2-butanol vom Schmelzpunkt 177 °C bis 178 °C.

Beispiel 2

Herstellung des Ausgangsproduktes

a)

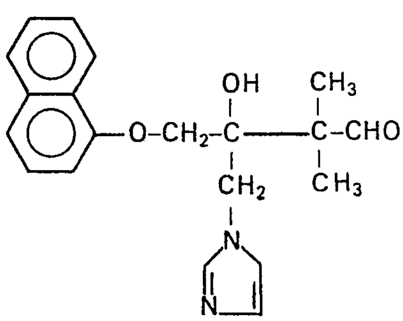

22,7 g (0,059 Mol) 3-(1,3-Dioxolan -2-yl) -2-(imidazol -1-yl-methyl) -3-methyl -1-(naphth -1-yl-oxy) -2-butanol in 150 ml Ethanol und 150 ml Wasser werden mit 15 ml konzentrierter Salzsäure versetzt und 4 Stunden unter Rückfluss erhitzt. Anschliessend wird das Reaktionsgemisch in gesättigte, wässrige Natriumhydrogencarbonat-Lösung gegossen und dreimal mit Methylenchlorid extrahiert. Die vereinigten organischen Phasen werden mit Wasser gewaschen, über Natriumsulfat getrocknet und eingeengt. Der Rückstand wird in Ether/Essigester aufgenommen und abgesaugt.

Man erhält 17,0 g (85,3% der Theorie) 2,2-Dimethyl-3-hydroxy -3-(imidazol -1-yl-methyl) -4-(naphth -1-oxy)-butanal vom Schmelzpunkt 147 °C.

b)

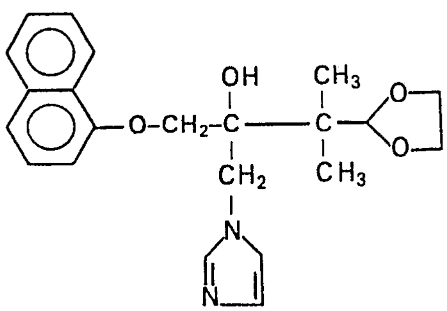

Eine Lösung von 53,5 g (0,17 Mol) 2-[2-(1,3-Dioxolan -2-yl)-prop -2-yl] -2-(naphth -1-yl-oxymethyl)-oxiran, 12,8 g (0,188 Mol) Imidazol und 1,3 g Kaliumhydroxid in 350 ml absolutem Butanol wird 16 Stunden unter Rückfluss erhitzt. Man lässt auf Raumtemperatur abkühlen und versetzt mit 500 ml Methylenchlorid. Das Reaktionsgemisch wird zweimal mit Wasser gewaschen. Die organische Phase wird abgetrennt, über Natriumsulfat getrocknet und eingeengt. Der Rückstand wird mit 350 ml Diisopropylether und Essigester versetzt. Der ausgefallene Niederschlag wird abgesaugt.

Man erhält 38,3 g (59,4% der Theorie) 3-(1,3-Dioxolan-2-yl) -2-(imidazol -1-yl-methyl) -3-methyl -1-(naphth -1-yloxy) -2-butanol vom Schmelzpunkt 126 °C.

c)

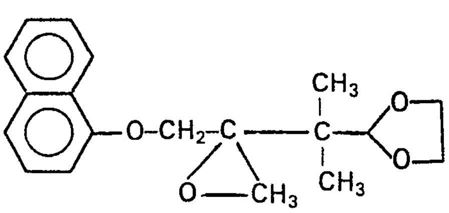

131,1 g (0,596 Mol) Trimethylsulfoxoniumiodid in 120 ml absolutem Dimethylformamid werden portionsweise mit 67,2 g (0,6 Mol) Kalium-tert.-butylat versetzt. Man lässt 6 Stunden bei Raumtemperatur nachrühren und versetzt dann mit einer Lösung von 122 g (0,407 Mol) [2-(1,3-Dioxolan -2-yl)-prop-2-yl]-(naphth -1-yl-oxymethyl)-keton in 550 ml absolutem Tetrahydrofuran. Die Reaktionsmischung wird über Nacht bei Raumtemperatur nachgerührt, eingeengt, in Methylenchlorid aufgenommen, zweimal mit Wasser gewaschen, über Natriumsulfat getrocknet und eingeengt. Der Rückstand wird in Petrolether ausgerührt und abgesaugt.

Man erhält 107 g (83,7% der Theorie) vom Schmelzpunkt 61 °C.

d)

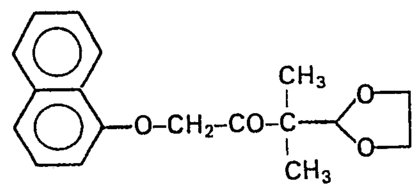

Eine Lösung von 141,5 g (0,735 Mol) 1-Chlor-3-(1,3-dioxolan-2-yl) -3-methyl -2-butanon, 105,9 g (0,835 Mol) 1-Naphthol und 122 g (0,882 Mol) Kaliumcarbonat in 1000 ml absolutem Ethyl-methyl-keton wird 16 Stunden unter Rückfluss erhitzt. Man lässt auf Raumtemperatur abkühlen und filtrieren. Das Filtrat wird eingeengt, mit Methylenchlorid versetzt, einmal mit verdünnter Natronlauge und zweimal mit Wasser gewaschen, über Natriumsulfat getrocknet und eingeengt. Der Rückstand wird in Petrolether ausgerührt, abgesaugt und getrocknet.

Man erhält 122,6 g (55,6% der Theorie [2-(1,3-Dioxolan -2-yl)-prop -2-yl]-(naphth -1-yl-oxymethyl)-keton vom Schmelzpunkt 69 °C.

e)

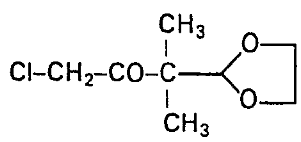

204 g (1,38 Mol) 4-Chlor-2,2-dimethyl -3-keto-butanol werden mit 93 g (1,5 Mol) Ethylenglykol und 0,7 g p-Toluolsulfonsäure in 400 ml Methylenchlorid während 3 Stunden am Wasserabscheider erhitzt. Die organische Phase wird mit 150 ml 5%iger Natronlauge und danach mit 400 ml Wasser extrahiert. Das Lösungsmittel wird abdestilliert und der Rückstand im Wasserstrahlvakuum destilliert.

Man erhält 211 g (79,8% der Theorie) 1-Chlor-3-(1,3-dioxolan -2-yl) -3-methyl-butan -2-on vom Siedepunkt 127 °C bis 128 °C/14 mbar.

f)

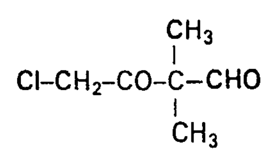

210 g (1,5 Mol) 1-(N-Morpholino)-isobuten werden innerhalb einer Stunde in 169 g (1,5 Mol) Chloracetylchlorid, gelöst und 350 ml Diethylether, bei 5 °C zugetropft. Nach beendeter Zugabe rührt man weitere 3 Stunden unter Rückflusskühlung. Die Lösung wird auf 100 g Eis gegossen, mit wässriger Natriumhydrogencarbonatlösung auf pH 5 gebracht und die Etherphase abgetrennt. Die Wasserphase wird mit 100 ml Diethylether extrahiert, die organischen Phasen werden vereinigt, über Natriumsulfat getrocknet, das Lösungsmittel abdestilliert und der Rückstand im Wasserstrahlvakuum destilliert.

Man erhält 136,4 g (61% der Theorie) 4-Chlor-2,2-dimethyl -3-keto-butanol vom Siedepunkt 95 °C bis 98 °C/14 mbar.

In analoger Weise und entsprechend den erfindungsgemässen Verfahrensangaben werden die nachfolgenden Aldehydvorstufen der Formel

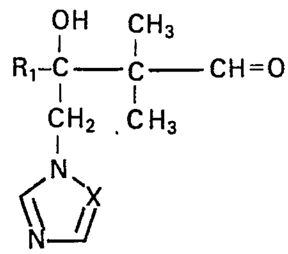

erhalten.

Tabelle 1

| Beisp. Nr. | $R^1$ | X | Schmelzpunkt (°C) bzw. spektroskopische Daten |
|---|---|---|---|
| 3 | 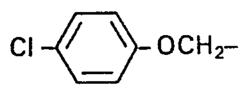 | N | 107 |
| 5 | 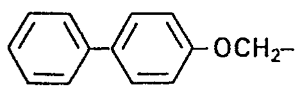 | N | Öl/$IR_{CHCl3}$:CHO = 1720 $cm^{-1}$ |
| 6 | 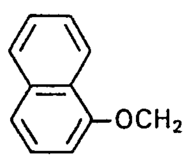 | N | Öl/$IR_{CHCl3}$:CHO = 1722 $cm^{-1}$ |
| 7 | 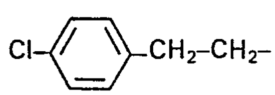 | N | 105 |
| 8 | 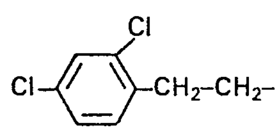 | N | 136 |
| 9 | 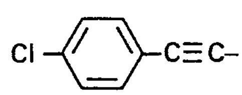 | N | 119 |
| 11 | 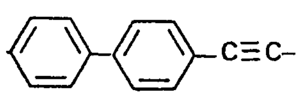 | N | 147 |
| 14 | 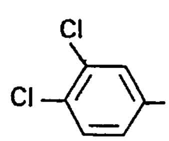 | N | 147–151 |
| 16 | 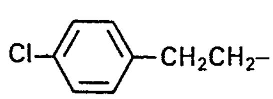 | CH | 127 |
| 19 | 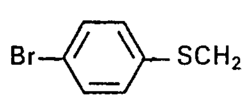 | N | Öl/$IR_{CHCl3}$:CHO = 1720 $cm^{-1}$ |

In analoger Weise und entsprechend den erfindungsgemässen Verfahrensangaben werden die nachfolgenden Verbindungen der allgemeinen Formel (I) erhalten:

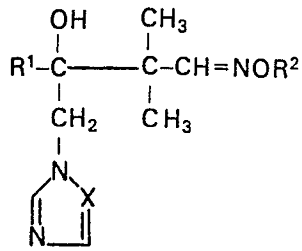

(I)

Tabelle 2

| Beisp. Nr. | $R^1$ | X | | Schmelzpunkt (°C) bzw. spektroskopische Daten |
|---|---|---|---|---|
| 4 | 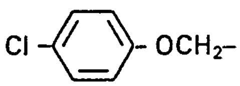 | N | $CH_3$ | 144 |
| 10 | 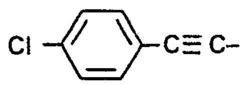 | N | $CH_3$ | 121 |
| 12 | 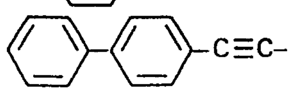 | N | $CH_3$ | 151 |
| 13 | 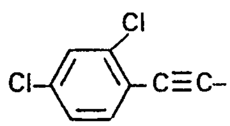 | N | $CH_3$ | 93 |
| 15 | 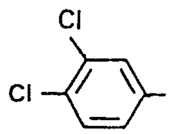 | N | $CH_3$ | 96–101 |
| 17 | 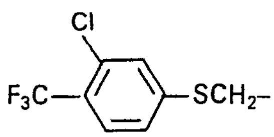 | N | $CH_3$ | Öl/$^1$H-NMR$_{CDCl_3}$: = 4,45 ppm für –C $H_2$ – 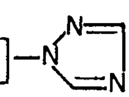 |
| 18 | 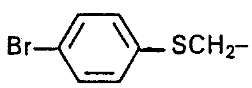 | N | $CH_3$ | Öl/$^1$H-NMR$_{CDCl_3}$: = 4,42 ppm für –C $H_2$ – 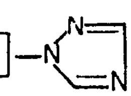 |
| 20 | 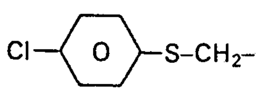 | CH | $CH_3$ | Öl/$^1$H-NMR$_{CDCl_3}$: = 4,13 ppm für –C $H_2$ – 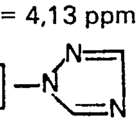 |
| 21 | 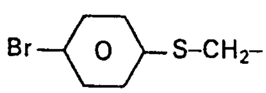 | CH | $CH_3$ | Öl/$^1$H-NMR$_{CDCl_3}$: = 4,15 ppm für –C $H_2$ – 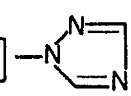 |
| 22 | 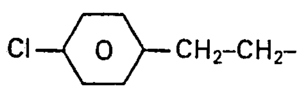 | N | $CH_3$ | Öl/$^1$H-NMR$_{CDCl_3}$: = 4,35 ppm für –C $H_2$ – 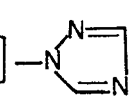 |
| 23 | 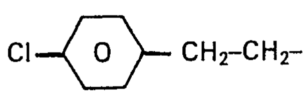 | N | $C_4H_9$ | Öl/$^1$H-NMR$_{CDCl_3}$: = 4,35 ppm für –C $H_2$ – 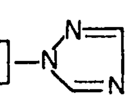 |

| Beisp. Nr. | $R^1$ | X | $R^2$ | Schmelzpunkt (°C) bzw. spektroskopische Daten |
|---|---|---|---|---|
| 24 | 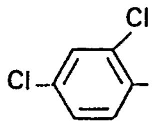 $O-CH_2$ | N | $CH_3$ | 119 |
| 25 | 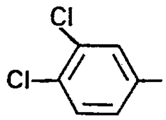 | N | $C_2H_5$ | 127–129 |
| 26 | 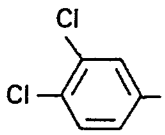 | N | $C_4H_9$-n | 138–140 |
| 27 | 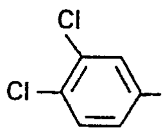 | N | $CH_2-CH=CH_2$ | 140–142 |
| 28 | 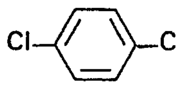 $CH_2CH_2-$ | N | $CH_2$ 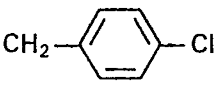 | 125–127 |
| 29 | 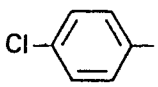 $CH_2CH_2-$ | N | $CH_2$ 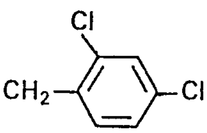 | 97 |
| 30 | 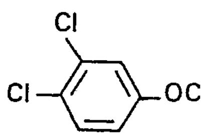 $OCH_2$ | N | $CH_2$ 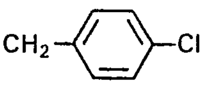 | 89– 90 |
| 31 | 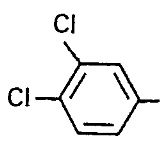 $OCH_2$ | N | $CH_2$ 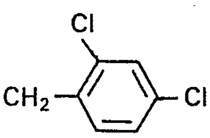 | 72 |
| 32 | 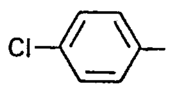 $SCH_2-$ | N | $CH_3$ | zähes Öl |
| 33 | 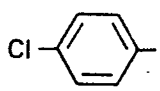 $SCH_2-$ | N | $CH_2-CH=CH_2$ | 100–101 |
| 34 | $CH_3$ | N | $CH_2$ 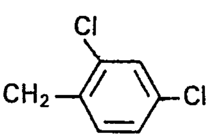 | zähes Öl |
| 35 | 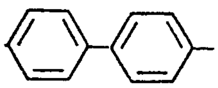 | N | $CH_3$ | 55– 57 |
| 36 | 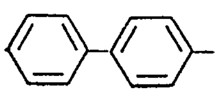 | N | $CH_2-CH=CH_2$ | 75 |
| 37 | 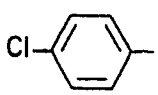 | N | $CH_3$ | 96–101 |

Tabelle 2

| Beisp. Nr. | $R^1$ | X | $R^2$ | Schmelzpunkt (°C) bzw. spektroskopische Daten |
|---|---|---|---|---|
| 38 | 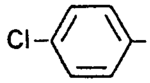 | N | $C_3H_7$–n | 112–114 |
| 39 | 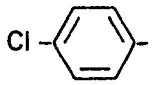 | N | 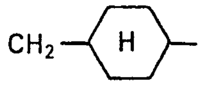 | 130–133 |
| 40 | 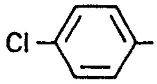 | N | $C_4H_9$–n | 124–130 |
| 41 | $C_3H_7$–n– | N | 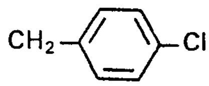 | zähes Öl |
| 42 | $C_3H_7$–n– | N | 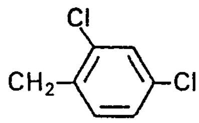 | zähes Öl |
| 43 | $C_2H_5$– | N | 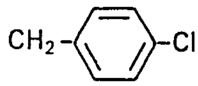 | $n_D^{20} = 1{,}5456$ |
| 44 | $C_2H_5$– | N | 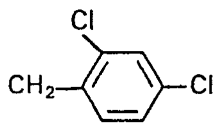 | zähes Öl |
| 45 | $C_4H_9$–n– | N | 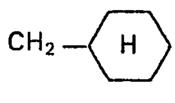 | $n_D^{20} = 1{,}5021$ |
| 46 | $C_4H_9$–n– | N | 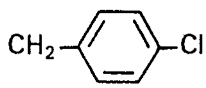 | $n_D^{20} = 1{,}5353$ |
| 47 | $C_4H_9$–n– | N | 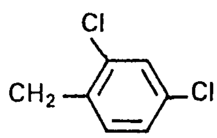 | $n_D^{20} = 1{,}5438$ |
| 48 | $CH_3$–$(CH_2)_4$– | N | $CH_3$ | 66– 68 |
| 49 | $CH_3$–$(CH_2)_2$– | N | $CH_3$ | 91 |
| 50 | $CH_3$–$(CH_2)_2$– | N | $C_3H_7$–n | $n_D^{20} = 1{,}4886$ |
| 51 | $CH_3$–$(CH_2)_2$– | N | 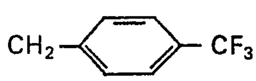 | 54– 57 |
| 52 | $CH_3$–$(CH_2)_4$– | N | $C_3H_7$–n | $n_D^{20} = 1{,}4865$ |
| 53 | $CH_3$–$(CH_2)_4$– | N | $CH_2$–CH=$CH_2$ | $n_D^{20} = 1{,}4949$ |
| 54 | $CH_3$–$(CH_2)_4$– | N | 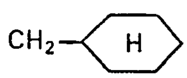 | $n_D^{20} = 1{,}4953$ |

Tabelle 2

| Beisp. Nr. | $R^1$ | X | $R^2$ | Schmelzpunkt (°C) bzw. spektroskopische Daten |
|---|---|---|---|---|
| 55 | $CH_3-(CH_2)_4-$ | CH | 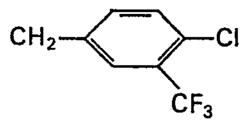 | zähes Öl |
| 56 | $CH_3-(CH_2)_4-$ | CH | 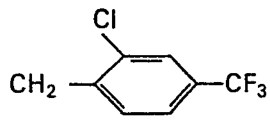 | zähes Öl |
| 57 | $CH_3-(CH_2)_4-$ | CH | 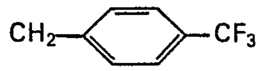 | zähes Öl |
| 58 | $CH_3-(CH_2)_4-$ | CH | $CH_3$ | zähes Öl |
| 59 | 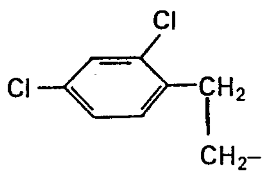 | N | $CH_3$ | zähes Öl |
| 60 | 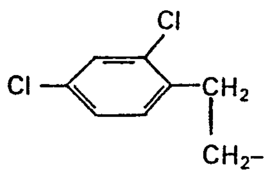 | N | $C_2H_5$ | zähes Öl |
| 61 | 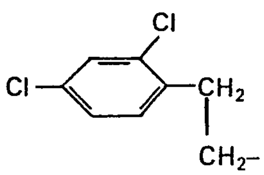 | N | $C_3H_7$-n | zähes Öl |
| 62 | 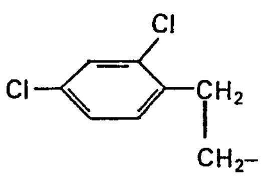 | N | $CH_2-CH=CH_2$ | zähes Öl |
| 63 | 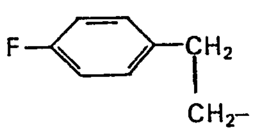 | N | $CH_3$ | zähes Öl |
| 64 | 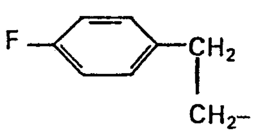 | N | $C_2H_5$ | zähes Öl |
| 65 | 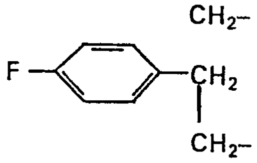 | N | $C_3H_7$-n | zähes Öl |
| 66 | 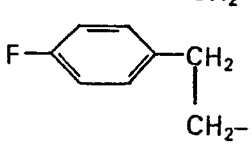 | N | $C_4H_9$-n | zähes Öl |

Tabelle 2

| Beisp. Nr. | $R^1$ | X | $R^2$ | Schmelzpunkt (°C) bzw. spektroskopische Daten |
|---|---|---|---|---|
| 67 | 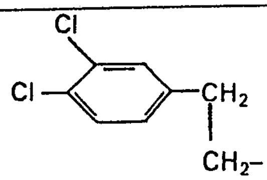 | N | $CH_3$ | 78–80 |
| 68 | 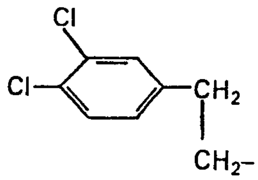 | N | $C_3H_7$–n | zähes Öl |
| 69 | 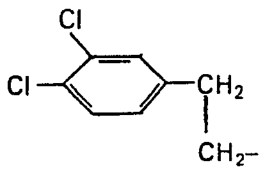 | N | $C_4H_9$–n | zähes Öl |
| 70 | 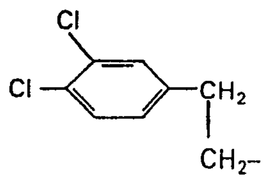 | N | $CH_2$–CH=$CH_2$ | zähes Öl |
| 71 | $CH_3$–$(CH_2)_3$– | N | $CH_3$ | $n_D^{20}$ = 1,4940 |
| 72 | $CH_3$–$(CH_2)_3$– | N | $C_3H_7$–n | $n_D^{20}$ = 1,4865 |
| 73 | $CH_3$–$(CH_2)_3$– | N | $CH_2$–CH=$CH_2$ | 60– 62 |
| 74 | $CH_3$–$(CH_2)_3$– | N | 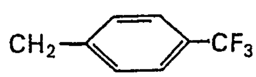 | $n_D^{20}$ = 1,4974 |
| 75 | $CH_3$–$(CH_2)_3$– | N | 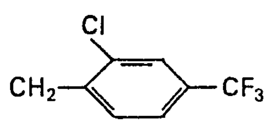 | $n_D^{20}$ = 1,5060 |
| 76 | 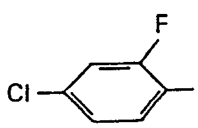 | N | $CH_3$ | 144 |
| 77 | 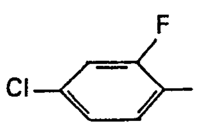 | N | $C_3H_7$–n | 130– 32 |
| 78 | 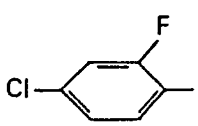 | N | $C_2H_5$ | 134– 37 |

Tabelle 2

| Beisp. Nr. | $R^1$ | X | $R^2$ | Schmelzpunkt (°C) bzw. spektroskopische Daten |
|---|---|---|---|---|
| 79 | 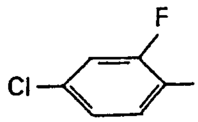 | N | $C_4H_9$-n | 144– 46 |
| 80 | 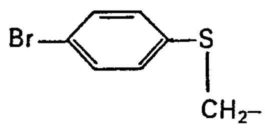 | CH | $C_3H_7$-n | zähes Öl |

Verwendungsbeispiele

In den nachfolgenden Verwendungsbeispielen werden die nachstehend angegebenen aus DE-OS 3 018 865 bekannten Verbindungen als Vergleichsverbindungen eingesetzt:

(A)

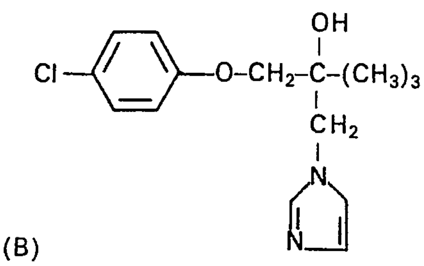

(B)

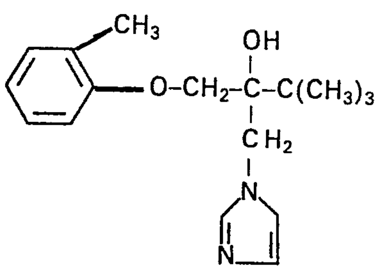

Beispiel A

Antimykotische in-vitro-Wirksamkeit

Versuchsbeschreibung:

Die in-vitro-Prüfungen wurden im Reihenverdünnungstest mit Keiminokula von durchschnittlich $5 \times 10^3$ bis $10^4$ Keimen/ml Substrat durchgeführt. Als Nährmedium dienten

a) für Dermatophyten und Schimmelpilze: Sabouraud's milieu d'épreuve

b) für Hefen: Fleischextrakt-Traubenzucker-Bouillon.

Die Bebrütungstemperatur betrug 28 °C bis 37 °C, die Bebrütungsdauer lag bei 24 bis 96 Stunden bei Hefen und 96 Stunden bei Dermatophyten und Schimmelpilzen.

In diesem Test zeigten z.B. die erfindungsgemässen Verbindungen 4, 10, 12, 13, 17, 18, 20, 21, 22, 23, 28, 29, 30, 32, 33, 35, 36, 37, 38, 41, 42, 43, 45, 46 und 47 eine bessere antimykotische Wirkung als die aus dem Stand der Technik bekannte Verbindung (B).

Tabelle A

Antimykotische in-vitro-Wirksamkeit

| Wirkstoff | MHK-Werte in ml Nährmedium Tricha-phyton mentagr. | Micro-sporum canis | Candida albi-cans | Toru-lopsis glabrata | Asper-gillus fumigauts |
|---|---|---|---|---|---|
| (B) (bekannt) | 32 | – | 32 | >64 | >64 |
| Verbindungen gemäss Herstellungsbeispiel: | | | | | |
| 4 | <1 | 4 | <1 | 32 | 16 |
| 10 | <1 | 4 | 8 | 32 | 4 |
| 12 | <1 | 2 | <1 | 32 | 2 |
| 13 | <1 | 4 | 2 | 32 | 32 |
| 17 | <1 | 4 | 16 | 64 | 4 |
| 18 | <1 | 4 | 4 | 4 | 4 |
| 20 | <1 | 8 | <1 | <1 | 4 |
| 21 | <1 | 4 | <1 | <1 | 2 |

Tabelle A
Antimykotische in-vitro-Wirksamkeit

| Wirkstoff | MHK-Werte in ml Nährmedium Tricha-phyton mentagr. | Micro-sporum canis | Candida albi-cans | Toru-lopsis glabrata | Asper-gillus fumigauts |
|---|---|---|---|---|---|
| 22 | <1 | <1 | 8 | 8 | <1 |
| 23 | <1 | 8 | 16 | 4 | 4 |
| 28 | <1 | 32 | <1 | 2 | 2 |
| 29 | <1 | 64 | <1 | 16 | 16 |
| 30 | <1 | 64 | 2 | 16 | 16 |
| 32 | <1 | 16 | <1 | <1 | 2 |
| 33 | <1 | 64 | <1 | <1 | 4 |
| 35 | <1 | 4 | <1 | 8 | 4 |
| 36 | <1 | 16 | 8 | 4 | 8 |
| 37 | <1 | 16 | <1 | 16 | 32 |
| 38 | <1 | 32 | <1 | 4 | 32 |
| 41 | <1 | 4 | 2 | 64 | 4 |
| 42 | 2 | 4 | 8 | 32 | 8 |
| 43 | <1 | 16 | 16 | 64 | 4 |
| 45 | 4 | 16 | <1 | 4 | 8 |
| 46 | 2 | 4 | <1 | 8 | 8 |
| 47 | 4 | 8 | <1 | 16 | 8 |

Beispiel B
Antimykotische in-vivo-Wirksamkeit (oral) bei Mäusecandidose
Versuchsbeschreibung:

Mäuse vom Typ SPF-$CF_1$ wurden intravenös mit $1-2 \times 10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 25–100 mg/kg Körpergewicht der Präparate oral behandelt.

Ergebnis:

Unbehandelte Tiere starben 3 bis 6 Tage post infektionem. Die Überlebensrate am 6. Tag post infektionem betrug bei unbehandelten Kontrolltieren etwa 5%.

In diesem Test zeigen z.B. die erfindungsgemässen Verbindungen 4, 10, 12, 13, 18, 20, 23, 24, 28, 32, 37, 38, 39 und 40 eine bessere Wirkung als die aus dem Stand der Technik bekannten Verbindungen (A) und (B).

Zeichenerklärung:

+++++ = sehr gute Wirkung = 90% Überlebende am 6. Tag p.i.
++++ = gute Wirkung = 80% Überlebende am 6. Tag p.i.
+++ = Wirkung = 60% Überlebende am 6. Tag p.i.
++ = schwache Wirkung = 40% Überlebende am 6. Tag p.i.
+ = Spur Wirkung = unter 40% Überlebende am 6. Tag p.i.
k.W. = keine Wirkung = kein Unterschied zur unbehandelten Infektionskontrolle

Tabelle B
Antimykotische in-vivo-Wirkung (oral) bei Mäusecandidose

| Wirkstoff | Wirkung |
|---|---|
| (A) (bekannt) | k. W. |
| (B) (bekannt) | k. W. |
| Verbindungen gemäss Herstellungsbeispiel: | |
| 4 | +++++ |
| 10 | +++++ |
| 12 | ++++ |
| 13 | +++ |
| 18 | +++++ |
| 20 | ++++ |
| 22 | +++++ |
| 23 | +++ |
| 24 | +++ |
| 28 | +++ |
| 32 | +++++ |
| 37 | +++++ |
| 38 | +++++ |
| 39 | ++++ |
| 40 | +++++ |

Beispiel C
Antimikrobielle in-vivo-Wirksamkeit (lokal) am Modell der experimentellen Meerschweinchen-Trichophytie
Versuchsbeschreibung:

Weisse Mäuse der Rasse Pirbright-white wurden auf dem geschorenen, nicht skarifizierten Rücken mit einer Mikro- und Makrokonidien-Suspension von Trichophyton mentagrophytes infiziert.

Die infizierten Tiere wurden beginnend mit dem 3. Tag p.i. 1 ×täglich mit einer 0,1%igen Lösung der erfindungsgemässen Präparate (in Dimethylsulfoxid:Glycerin = 1:4) lokal behandelt.

Ergebnis:

Bei unbehandelten Tieren entwickelte sich innerhalb 12 Tagen p.i. das typische Bild einer Dermtophytose mit Rötung, Schuppung und Haarausfall bis zum totalen Integument-Defekt an der Infektionsstelle.

In diesem Test zeigen z.B. die erfindungsgemässen Verbindungen 18, 20, 22, 32, 37, 38 und 40 Wirkung bis gute Wirkung.

Tabelle C
Antimykotische in-vivo-Wirksamkeit (lokal) am Modell der experimentellen Meerschweinchen-Trichophytie

| Wirkstoff | Wirkung |
|---|---|
| Verbindungen gemäss Herstellungsbeispiel: | |
| 18 | +++ |
| 20 | +++ |
| 22 | +++ |
| 32 | ++++ |
| 37 | ++++ |
| 38 | ++++ |
| 40 | +++ |

Erläuterungen:

| | | |
|---|---|---|
| +++++ | = sehr gute Wirkung | = keine Infektionszeichen am 12. bis 15. Tag p.i |
| ++++ | = gute Wirkung | = geringe Rötung, vereinzelt Schuppen |
| +++ | = Wirkung | = Rötung, Schuppen ohne Haarausfall |
| ++ | = schwache Wirkung | = Rötung, Schuppen, Haarausfall |
| + | = Spur Wirkung | = flächiger Haarausfall, entzündliche Hautreaktion |

Beispiel/Formulierungen

1. Lösung:

| | | |
|---|---|---|
| Wirkstoff gemäss Formel (I) | : | 10 g |
| Alkohol, rein (96%ig) | : | 300 g |
| Isopropylmyristat | : | 526 g |
| | | 836 g |

2. Creme:

| | | |
|---|---|---|
| Wirkstoff gemäss Formel (I) | : | 10 g |
| Arlacel 60 | : | 20 g |
| (Sorbitan-monostearat) Tween 60 | : | 15 g |
| (Polyoxyethylen (20)-sorbitanmonostearat) Walrat, künstlich | : | 30 g |
| (Mischung von Estern von gesättigten Fettsäuren $C_{14}$–$C_{18}$ und Fettalkoholen $C_{14}$–$C_{18}$) Lanette O | : | 100 g |
| (Gemisch aus Cetyl-Alkohol und Stearyl-Alkohol) Etanol G | : | 135 g |
| (2-Octyl-dodecanol) Benzylalkohol | : | 10 g |
| Wasser, entmineralisiert | : | 680 g |
| | | 1000 g |

## Patentansprüche

1. Hydroxyethyl-azol-Derivate der allgemeinen Formel

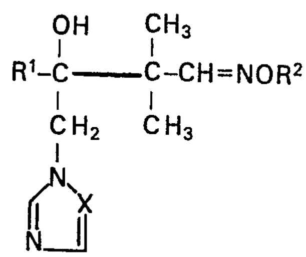

(I)

in welcher

$R^1$ für geradkettiges oder verzweigtes Alkyl mit 1 bis 6 Kohlenstoffatomen oder für die Gruppierung Ar–Y;

Ar für Naphthyl oder für gegebenenfalls einfach oder mehrfach, gleich oder verschieden substituiertes Phenyl, wobei als Substituenten genannt seien:

Halogen, Alkyl mit 1 bis 4 Kohlenstoffatomen, Alkoxy und Alkylthio mit jeweils 1 bis 2 Kohlenstoffatomen, Nitro, Halogenalkyl und Halogenalkoxy sowie Halogenalkylthio mit jeweils 1 bis 2 Kohlenstoffatomen und 1 bis 5 gleichen oder verschiedenen Halogenatomen, wie vorzugsweise Fluor und Chloratomen, der $-CH=NOR^2$-Rest, sowie jeweils gegebenenfalls durch Halogen und/oder Alkyl mit 1 bis 2 Kohlenstoffatomen

substituiertes Phenyl, Phenoxy, Benzyl und Benzyloxy;

X für ein Stickstoffatom oder die CH-Gruppe;

Y für eine direkte Bindung oder für die Gruppierungen $-CH_2-$, $-CH_2CH_2-$, $-OCH_2-$, $-SCH_2-$, $-CH=CH-$ oder $-C\equiv C-$;

und

$R^2$ für Wasserstoff, geradkettiges oder verzweigtes Alkyl mit 1 bis 6 Kohlenstoffatomen, Alkenyl und Alkinyl mit jeweils 2 bis 6 Kohlenstoffatomen, für gegebenenfalls einfach oder mehrfach, gleich oder verschieden substituiertes Phenylalkyl mit 1 bis 2 Kohlenstoffatomen im Alkylteil, wobei als Phenylsubstituenten die bei Ar bereits genannten Phenylsubstituenten infrage kommen; sowie für gegebenenfalls einfach bis dreifach, gleich oder verschieden durch Alkyl mit 1 bis 3 Kohlenstoffatomen substituiertes Cycloalkylmethyl mit 5 oder 6 Kohlenstoffatomen im Cycloalkylteil steht und deren Säureadditions-Salze zur Verwendung bei der Behandlung von Mykosen.

2. Hydroxyethyl-azol-Derivate der allgemeinen Formel (I) in Anspruch 1,

in welcher

$R^1$ für geradkettiges Alkyl mit 1 bis 6 Kohlenstoffatomen oder für die Gruppierung Ar–Y– steht;

Ar für Naphthyl oder für gegebenenfalls einfach bis dreifach, gleich oder verschieden substituiertes Phenyl steht, wobei als Substituenten genannt seien:

Fluor, Chlor, Methyl, Trifluormethyl, Trifluormethoxy, Trifluormethylthio, Methoximinomethyl, Ethoximinomethyl, Allyloximinomethyl, sowie jeweils gegebenenfalls durch Chlor und/oder Methyl substituiertes Phenyl, Phenoxy, Benzyl und Benzyloxy;

X für ein Stickstoffatom oder die CH-Gruppe steht;

Y für eine direkte Bindung oder für die Gruppierungen $-CH_2-$, $-CH_2CH_2-$, $-OCH_2-$, $-SCH_2-$, $-CH=CH-$ oder $-C\equiv C-$ steht;

und

$R^2$ für Wasserstoff, Methyl, Ethyl, n-Propyl, n-Butyl, Allyl, Propargyl, für gegebenenfalls einfach bis dreifach, gleich oder verschieden durch Fluor, Chlor, Methyl, Trifluormethyl oder Trifluormethoxy substituiertes Benzyl sowie gegebenenfalls durch Methyl oder Ethyl substituiertes Cyclohexylmethyl steht und deren Säureadditions-Salze zur Verwendung bei der Behandlung von Mykosen.

3. Verbindung der Formel

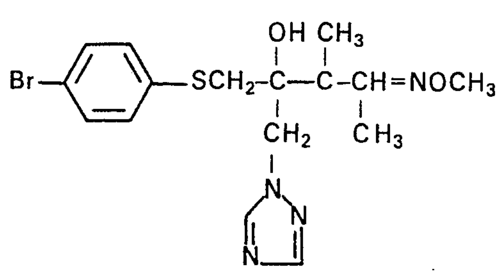

zur Verwendung bei der Bekämpfung von Mykosen.

4. Verbindung der Formel

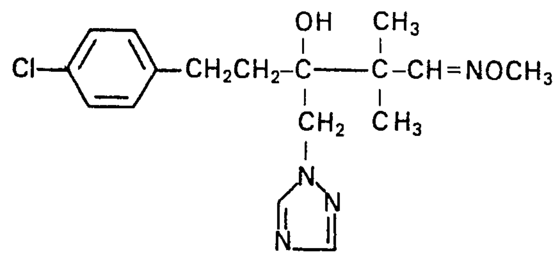

zur Verwendung bei der Bekämpfung von Mykosen.

5. Verbindung der Formel

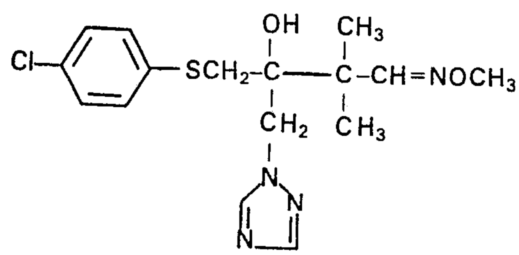

zur Verwendung bei der Bekämpfung von Mykosen.

6. Verbindung der Formel

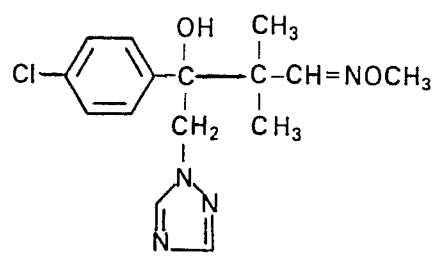

zur Verwendung bei der Behandlung von Mykosen:

7. Verbindung der Formel

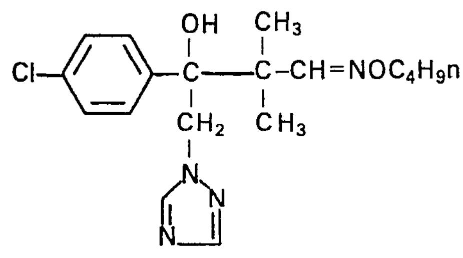

zur Verwendung bei der Behandlung von Mykosen.

8. Verwendung der Verbindungen der allgemeinen Formel (I) in Anspruch 1 zur Herstellung von Arzneimitteln zur Bekämpfung von Mykosen.

9. Arzneimittel enthaltend mindestens eine Verbindung der allgemeinen Formel (I) gemäss Anspruch 1.

10. Verfahren zur Herstellung von Arzneimitteln, dadurch gekennzeichnet, dass man Verbindungen der allgemeinen Formel I

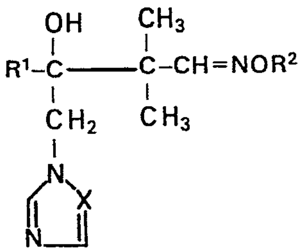

(I)

in welcher

$R^1$ für geradkettiges oder verzweigtes Alkyl mit 1 bis 6 Kohlenstoffatomen oder für die Gruppierung Ar–Y;

Ar für Naphthyl oder für gegebenenfalls einfach oder mehrfach, gleich oder verschieden substituiertes Phenyl, wobei als Substituenten genannt seien:

Halogen, Alkyl mit 1 bis 4 Kohlenstoffatomen, Alkoxy und Alkylthio mit jeweils 1 bis 2 Kohlenstoffatomen, Nitro, Halogenalkyl und Halogenalkoxy sowie Halogenalkylthio mit jeweils 1 bis 2 Kohlenstoffatomen und 1 bis 5 gleichen oder verschiedenen Halogenatomen, wie vorzugsweise Fluor und Chloratomen, der $-CH=NOR^2$-Rest, sowie jeweils gegebenenfalls durch Halogen und/oder Alkyl mit 1 bis 2 Kohlenstoffatomen substituiertes Phenyl, Phenoxy, Benzyl und Benzyloxy;

X für ein Stickstoffatom oder die CH-Gruppe;

Y für eine direkte Bindung oder für die Gruppierungen $-CH_2-$, $-CH_2CH_2-$, $-OCH_2-$, $-SCH_2-$, $-CH=CH-$ oder $-C\equiv C-$;

und

$R^2$ für Wasserstoff, geradkettiges oder verzweigtes Alkyl mit 1 bis 6 Kohlenstoffatomen, Alkenyl und Alkinyl mit jeweils 2 bis 6 Kohlenstoffatomen, für gegebenenfalls einfach oder mehrfach, gleich oder verschieden substituiertes Phenylalkyl mit 1 bis 2 Kohlenstoffatomen im Alkylteil, wobei als Phenylsubstituenten die bei Ar bereits genannten Phenylsubstituenten infrage kommen; sowie für gegebenenfalls einfach bis dreifach, gleich oder verschieden durch Alkyl mit 1 bis 3 Kohlenstoffatomen substituiertes Cycloalkylmethyl mit 5 oder 6 Kohlenstoffatomen im Cycloalkylteil gegebenenfalls unter Verwendung von zusätzlichen Hilfs- und Trägerstoffen in eine geeignete Applikationsform überführt.

## Revendications

1. Dérivés hydroxyéthyl-azoliques de formule générale:

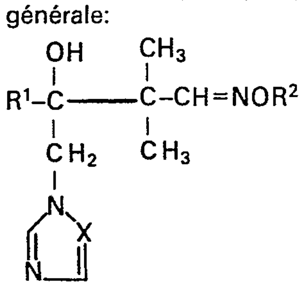

(I)

dans laquelle

$R^1$ est un groupe alkyle à chaîne droite ou ramifiée ayant 1 à 6 atomes de carbone ou le groupement Ar–Y;

Ar est un reste naphtyle ou un reste phényle portant éventuellement un ou plusieurs substituants identiques ou différents, avec comme substituants: halogène, alkyle ayant 1 à 4 atomes de carbone, alkoxy et alkylthio ayant chacun 1 ou 2 atomes de carbone, nitro, halogénalkyle et halogénalkoxy, ainsi qu'halogénalkylthio ayant chacun 1 ou 2 atomes de carbone et 1 à 5 atomes d'halogènes identiques ou différents, par exemple des atomes de fluor et de chlore, le reste $-CH=NOR^2-$ ainsi que phényle, phénoxy, benzyle et benzyloxy substitués chacun le cas échéant par un halogène et/ou un reste alkyle de 1 ou 2 atomes de carbone;

X est un atome d'azote ou le groupe CH;

Y est une liaison simple ou représente les groupements $-CH_2-$, $-CH_2CH_2-$, $-OCH_2-$, $-SCH_2-$, $-CH=CH-$ ou $-C\equiv C-$; et

$R^2$ est l'hydrogène, un groupe alkyle à chaîne droite ou à chaîne ramifiée ayant 1 à 6 atomes de carbone, un groupe alcényle et un groupe alcynyle ayant chacun 2 à 6 atomes de carbone, un groupe phénylalkyle portant éventuellement un ou plusieurs substituants identiques ou différents, ayant 1 ou 2 atomes de carbone dans la partie alkyle, et on considère alors comme substituants du groupe phényle les substituants du groupe phényle déjà mentionnés dans le cas de Ar; ainsi qu'un reste cycloalkylméthyle ayant 5 ou 6 atomes de carbone dans la partie cycloalkyle, et portant le cas échéant 1 à 3 substituants alkyle identiques ou différents, ayant 1 à 3 atomes de carbone; et leurs sels d'addition d'acides destinés à être utilisés dans le traitement de mycoses.

2. Dérivés hydroxyéthyl-azoliques de formule générale (I) suivant la revendication 1,

dans laquelle

$R^1$ est un groupe alkyle à chaîne droite ayant 1 à 6 atomes de carbone ou le groupement Ar–Y–;

Ar est un reste naphtyle ou un reste phényle portant éventuellement un à trois substituants identiques ou différents, et on mentionne alors comme substituants: le fluor, le chlore, un groupe méthyle, trifluorométhyle, trifluorométhoxy, trifluorométhylthio, méthoximinométhyle, éthoximinométhyle, allyloximinométhyle, des restes phényle, phénoxy, benzyle et benzyloxy substitués chacun le cas échéant par du chlore et/ou un groupe méthyle;

X est un atome d'azote ou le groupe CH;

Y est une liaison simple ou représente les groupements $-CH_2-$, $-CH_2CH_2-$, $-OCH_2-$, $-SCH_2-$, $-CH=CH$ ou $-C\equiv C-$;

et

$R^2$ est l'hydrogène, un groupe méthyle, éthyle, n-propyle, n-butyle, allyle, propargyle, un groupe benzyle portant éventuellement 1 à 3 substituants fluoro, chloro, méthyle, trifluorométhyle ou trifluorométhoxy identiques ou différents ainsi qu'un reste cyclohexylméthyle portant éventuellement un substituant méthyle ou éthyle et leurs

sels d'addition d'acides destinés à être utilisés dans le traitement de mycoses.

3. Composé de formule

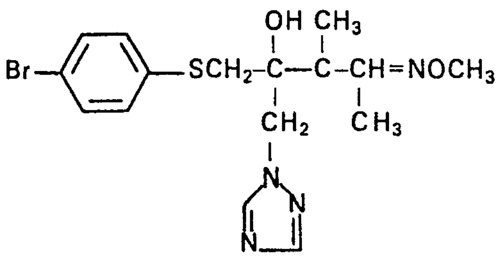

destiné à être utilisé dans le traitement de mycoses.

4. Composé de formule

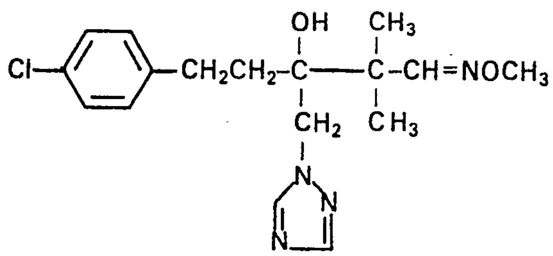

destiné à être utilisé pour combattre des mycoses.

5. Composé de formule

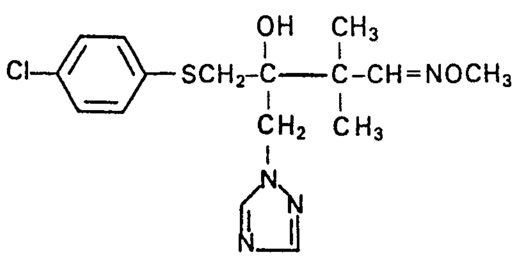

destiné à être utilisé pour combattre des mycoses.

6. Composé de formule

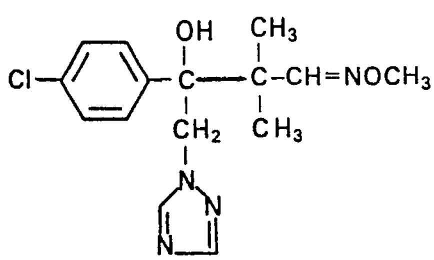

destiné à être utilisé pour combattre des mycoses.

7. Composé de formule

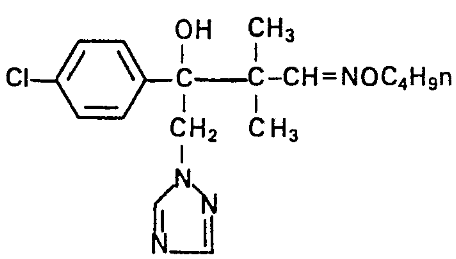

destiné à être utilisé pour combattre des mycoses.

8. Utilisation des composés de formule générale (I) suivant la revendication 1 pour la préparation de médicaments destinés à combattre des mycoses.

9. Médicaments contenant au moins un composé de formule générale (I) suivant la revendication 1.

10. Procédé de préparation de médicaments, caractérisé en ce qu'on incorpore dans une forme d'administration appropriée, le cas échéant en utilisant des additifs et des supports complémentaires, des composés de formule générale I

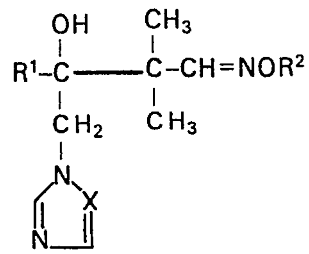

(I)

dans laquelle

$R^1$ est un groupe alkyle à chaîne droite ou ramifiée ayant 1 à 6 atomes de carbone ou le groupement Ar–Y;

Ar est un reste naphtyle ou un reste phényle portant éventuellement un ou plusieurs substituants identiques ou différents, avec comme substituants: halogène, alkyle ayant 1 à 4 atomes de carbone, alkoxy et alkylthio ayant chacun 1 ou 2 atomes de carbone, nitro, halogénalkyle et halogénalkoxy, ainsi qu'halogénalkylthio ayant chacun 1 ou 2 atomes de carbone et 1 à 5 atomes d'halogènes identiques ou différents, par exemple des atomes de fluor et de chlore, le reste $-CH=NOR^2-$ ainsi que phényle, phénoxy, benzyle et benzyloxy substitués chacun le cas échéant par un halogène et/ou un reste alkyle de 1 ou 2 atomes de carbone;

X est un atome d'azote ou le groupe CH;

Y est une liaison simple ou représente les groupements $-CH_2-$, $-CH_2CH_2-$, $-OCH_2-$, $-SCH_2-$, $-CH=CH-$ ou $-C\equiv C-$; et

$R^2$ est l'hydrogène, un groupe alkyle à chaîne droite ou à chaîne ramifiée ayant 1 à 6 atomes de carbone, un groupe alcényle et un groupe alcynyle ayant chacun 2 à 6 atomes de carbone, un groupe phénylalkyle portant éventuellement un ou plusieurs substituants identiques ou différents, ayant 1 ou 2 atomes de carbone dans la partie alkyle, et on considère alors comme substituants du groupe phényle les substituants du groupe phényle déjà mentionnés dans le cas de Ar; ainsi qu'un reste cycloalkylméthyle ayant 5 ou 6 atomes de carbone dans la partie cycloalkyle, et portant le cas échéant 1 à 3 substituants alkyle identiques ou différents, ayant 1 à 3 atomes de carbone;

## Claims

1. Hydroxyethyl-azole derivatives of the general formula

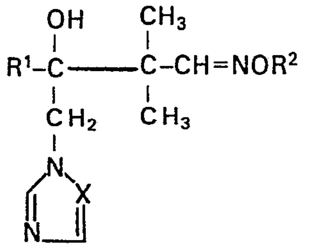 (I)

in which

$R^1$ represents straight-chain or branched alkyl with 1 to 6 carbon atoms or the grouping Ar–Y;

Ar represents naphthyl, or phenyl which is optionally monosubstituted or polysubstituted by identical or different substituents, substituents which may be mentioned being: halogen, alkyl with 1 to 4 carbon atoms, alkoxy and alkylthio with in each case 1 or 2 carbon atoms, nitro, halogenoalkyl, halogenoalkoxy and halogenoalkylthio with in each case 1 or 2 carbon atoms and 1 to 5 identical or different halogen atoms, such as, preferably, fluorine and chlorine atoms, the $-CH=NOR^2$ radical, and phenyl, phenoxy, benzyl and benzyloxy, each of which is optionally substituted by halogen and/or alkyl with 1 or 2 carbon atoms;

X represents a nitrogen atom or the CH group;

Y represents a direct bond or the groupings $-CH_2-$, $-CH_2CH_2-$, $-OCH_2-$, $-SCH_2-$, $-CH=CH-$ or $-C\equiv C-$;

and

$R^2$ represents hydrogen, straight-chain or branched alkyl with 1 to 6 carbon atoms, alkenyl or alkinyl with in each case 2 to 6 carbon atoms, or phenylalkyl which has 1 or 2 carbon atoms in the alkyl part and is optionally monosubstituted or polysubstituted by identical or different substituents, possible substituents on the phenyl being the substituents on phenyl which have already been mentioned in the case of Ar; or represents cycloalkylmethyl which has 5 or 6 carbon atoms in the cycloalkyl part and is optionally mono-, di- or tri-substituted by identical or different alkyl radicals with 1 to 3 carbon atoms, and acid addition salts thereof, for use in the treatment of mycoses.

2. Hydroxyethyl-azole derivatives of the general formula (I) in Claim 1, in which

$R^1$ represents straight-chain alkyl with 1 to 6 carbon atoms or the grouping Ar–Y–;

Ar represents naphthyl, or represents phenyl which is optionally mono-, di- or tri-substituted by identical or different substituents, substituents which may be mentioned being: fluorine, chlorine, methyl, trifluoromethyl, trifluoromethoxy, trifluoromethylthio, methoximinomethyl, ethoximinomethyl and allyloximinomethyl, and phenyl, phenoxy, benzyl and benzyloxy, each of which is optionally substituted by chlorine and/or methyl;

X represents a nitrogen atom or the CH group;

Y represents a direct bond or the groupings $-CH_2-$, $-CH_2CH_2-$, $-OCH_2-$, $-SCH_2-$, $-CH=CH-$ or $-C\equiv C-$; and

$R^2$ represents hydrogen, methyl, ethyl, n-propyl, n-butyl, allyl or propargyl, or represents benzyl which is optionally mono-, di- or tri-substituted by identical or different substituents from the group comprising fluorine, chlorine, methyl, trifluoromethyl and trifluoromethoxy, or represents cyclohexylmethyl which is optionally substituted by methyl or ethyl, and acid addition salts thereof, for use in the treatment of mycoses.

3. The compound of the formula

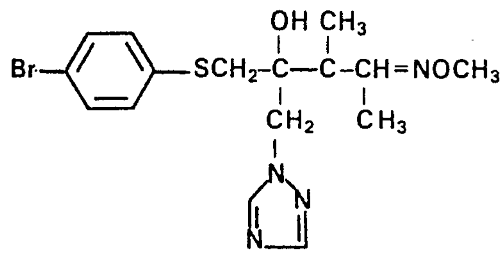

for use in combating mycoses.

4. The compound of the formula

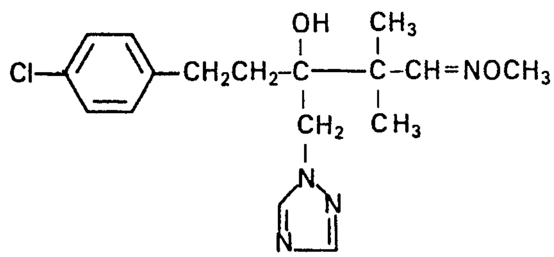

for use in combating mycoses.

5. The compound of the formula

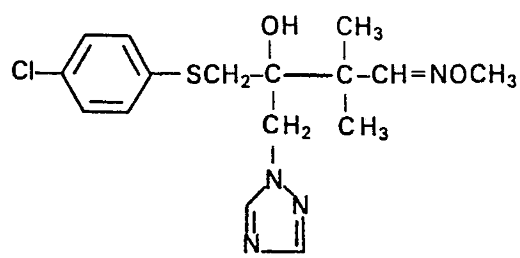

for use in combating mycoses.

6. The compound of the formula

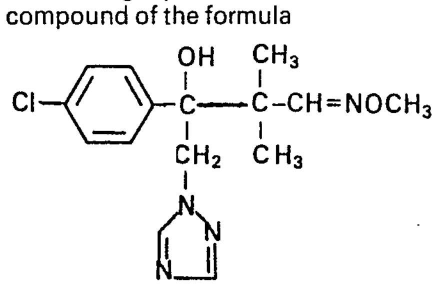

for use in the treatment of mycoses.

7. The compound of the formula

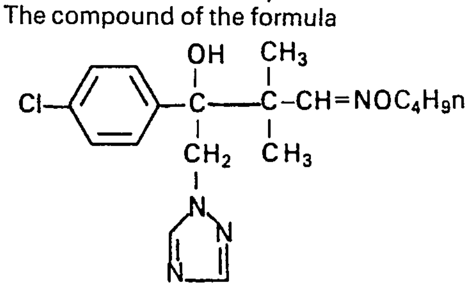

for use in the treatment of mycoses.

8. Use of the compounds of the general formula (I) in Claim 1 for the preparation of medicaments for combating mycoses.

9. Medicaments containing at least one compound of the general formula (I) according to Claim 1.

10. Process for the preparation of medicaments, characterized in that compounds of the general formula I

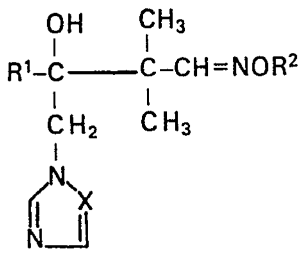

(I)

in which

$R^1$ represents straight-chain or branched alkyl with 1 to 6 carbon atoms or the grouping Ar–Y;

Ar represents naphthyl, or phenyl which is optionally monosubstituted or polysubstituted by identical or different substituents, substituents which may be mentioned being: halogen, alkyl with 1 to 4 carbon atoms, alkoxy and alkylthio with in each case 1 or 2 carbon atoms, nitro, halogenoalkyl, halogenoalkoxy and halogenoalkylthio with in each case 1 or 2 carbon atoms and 1 to 5 identical or different halogen atoms, such as, preferably, fluorine and chlorine atoms, the $-CH=NOR^2$ radical, and phenyl, phenoxy, benzyl and benzyloxy, each of which is optionally substituted by halogen and/or alkyl with 1 or 2 carbon atoms;

X represents a nitrogen atom or the CH group;

Y represents a direct bond or the groupings $-CH_2-$, $-CH_2CH_2-$, $-OCH_2-$, $-SCH_2-$, $-CH=CH-$ or $-C\equiv C-$;

and

$R^2$ represents hydrogen, straight-chain or branched alkyl with 1 to 6 carbon atoms, alkenyl or alkinyl with in each case 2 to 6 carbon atoms, or phenylalkyl which has 1 or 2 carbon atoms in the alkyl part and is optionally monosubstituted or polysubstituted by identical or different substituents, possible substituents on the phenyl being the substituents on phenyl which have already been mentioned in the case of Ar; or represents cycloalkylmethyl which has 5 or 6 carbon atoms in the cycloalkyl part and is optionally mono-, di- or tri-substituted by identical or different alkyl radicals with 1 to 3 carbon atoms, are converted into a suitable administration form optionally with the use of additional auxiliaries and excipients.